# EUROPEAN PATENT SPECIFICATION

(11) **EP 3 616 614 B1**
(45) Date of publication and mention of the grant of the patent: **04.01.2023**
(21) Application number: 18791314.0
(22) Date of filing: 16.03.2018
(51) Int. Cl.: A61B 5/157, A61B 5/155, A61B 5/151, A61B 5/15

(54) **INTEGRAL BLOOD GLUCOSE METER**
INTEGRIERTES BLUTZUCKERMESSGERÄT
GLUCOMÈTRE SANGUIN INTÉGRAL

(30) Priority: 24.04.2017 KR 20170052562
(43) Date of publication of application: 04.03.2020
(73) Proprietor: i-Sens, Inc., Seoul 06646 (KR)
(72) Inventor: CHAE, Kyung Chul, Seongnam-si Gyeonggi-do 13589 (KR); CHOI, Hyun Ho, Seoul 03097 (KR); RYU, Goang Yel, Goyang-si Gyeonggi-do 10518 (KR); WANG, Ji Hoon, Goyang-si Gyeonggi-do 10240 (KR); CHA, Geun Sig, Seoul 03610 (KR); NAM, Hak Hyun, Seoul 01227 (KR)
(74) Representative: Sander, Rolf
(86) International application number: PCT/KR2018/003128
(87) International publication number: WO 2018/199472

(56) References cited:
- WO-A2-02/078533
- JP-A- 2004 004 046
- JP-A- 2004 004 046
- KR-A- 20140 093 744
- KR-B1- 101 322 460
- US-A1- 2007 293 790
- US-A1- 2007 293 790
- US-A1- 2015 177 220
- US-B2- 8 961 432
- US-B2- 8 961 432

## Description

### TECHNICAL FIELD

The present disclosure relates to an integrated blood glucose meter. More particularly, the present disclosure relates to an integrated blood glucose meter having a simple operating method and structure, by which a strip cartridge, in which a plurality of test strips are stored, can be accommodated in the integrated blood glucose meter, and test strips can be loaded individually from the strip cartridge by a linear movement of an operating knob, so that test strips can be accurately supplied and discharged. A cartridge supply opening can be opened only while the operating knob is being manipulated, so that a function of hermetically closing the strip cartridge can be reliably maintained. A test strip can be prevented from degeneration even when used for an extended period of time, thereby improving the accuracy of a glucose measurement result.

### BACKGROUND ART

Diabetes or diabetes mellitus is a chronic medical condition that is common in modern people. In the Republic of Korea, it affects 2 million people, about 5% of the total population.

Diabetes is caused from the absolute deficiency or relative insufficiency of insulin produced by the pancreas, due to various causes such as obesity, stress, poor eating habits, inherited hereditary factors, in which glucose balance in the blood may be disturbed, thereby resulting in a high blood sugar level.

The blood usually contains a certain concentration of glucose, and tissue cells gain energy from the glucose.

However, when the content of glucose is increased excessively, it is not properly stored in the liver, muscle, or adipose tissue and accumulates in the blood. As a result, patients with diabetes maintain a much higher blood glucose level than other people. Excessive blood glucose passes through the tissues and is discharged into the urine, resulting in deficiency of glucose, which is absolutely necessary for all tissues of the body, thereby causing abnormalities in respective body tissues.

Diabetes mellitus is characterized by substantial absence of subjective symptoms at the beginning of the condition. When diabetes mellitus progresses, diabetes-specific symptoms such as diarrhea, polyuria, weight loss, general anxiety, skin itchiness, and scarring of the hands and feet are present. Further progression of diabetes leads to complications such as visual disturbances, hypertension, kidney disease, paralysis, periodontal disease, muscle spasms and neuralgia, as well as gangrene.

Systematic blood glucose measurement and treatment should be performed to diagnose diabetes beforehand and manage the condition to prevent the progression of diabetes into complications associated therewith.

For people with diabetes or those who have not yet developed diabetes, but have higher than normal blood glucose, medical device manufacturers offer a variety of blood glucose meters to measure blood glucose at home.

In such a blood glucose meter, a test strip is input to a container of the blood glucose meter, a blood sample is absorbed by the input test strip, a glucose level is measured by inspecting the absorbed blood sample using a glucose measuring module within the container, and a measurement result is calculated by a calculator and is output on a separate display.

To use such a blood glucose meter, a user should possess and carry a separate strip storage container in which a plurality of test strips are stored. A glucose measurement process is performed by taking one test strip from the strip storage container and inputting the test strip to the blood glucose meter, and after the glucose measurement process, the used test strip must be discharged and discarded, thereby making it inconvenient to use the blood glucose meter. In addition, since the strip storage container must be carried separately, there are significant difficulties in the use of the blood glucose meter.

To reduce or overcome such inconveniences in the use thereof, various types of integrated blood glucose meters have recently been developed, in each of which a strip cartridge storing a plurality of test strips can be accommodated in an internal space of the blood glucose meter, and test strips can be supplied individually from the strip cartridge.

However, such integrated blood glucose meters having been developed to date have a range of problems. For example, such integrated blood glucose meters may have a complicated structure or operating system, or test strips may not be efficiently supplied.

WO02078533A2 discloses an integrated sample testing meter, for instance for measuring blood, comprising a lancing device, an electrochemical sensor and a test strip cartridge disposed in a single modular housing. The integrated test system automatically dispenses and positions a test strip in proximity to a lancet puncture site, automatically transfers a blood sample to the test strip from the lancet puncture site and automatically analyzes the blood sample after the test strip collects the sample from the puncture site.

US2015177220 A1 discloses a measuring device comprising a main body which defines a storage space in the lengthwise direction, and in which a plurality of biosensors can be stacked; a cover member covering one end of the storage space and including a discharge hole through which only one biosensor located at the outermost edge of the stacked biosensors can pass; and a cover coupled to the cover member in order to be raised and lowered so as to open and close the discharge hole.

US2007293790A1 discloses that a diagnostic system has a diagnostic module with an integrated lancet system and a magazine module separate from the diagnostic module. The diagnostic device also has a connecting device that is used for tensioning the lancet system.

JP2004004046A discloses a biosensor cartridge allowing biosensors to be set one by one in a testable state by simple operation and protecting the biosensors from humidity until they are set, and to provide a biosensor allocator.

US8961432B2 discloses a method for implementing an actuator mechanism of an analyte testing device. In one aspect, a method to test an analyte includes advancing an analyte sensor from a first position within a sensor cartridge of an analyte testing device to a second position that exposes at least a portion of the analyte sensor outside of the device.

### DISCLOSURE

### Technical Problem

Accordingly, the present disclosure has been made in consideration of the above-described problems occurring in the related art, and an objective of the present disclosure is to provide an integrated blood glucose meter having a simple operating method and structure, by which a strip cartridge, in which a plurality of test strips are stored, can be accommodated in the integrated blood glucose meter, and test strips can be loaded individually from the strip cartridge by a linear movement of an operating knob, so that test strips can be accurately supplied and discharged.

Another objective of the present disclosure is to provide an integrated blood glucose meter, in which a cartridge supply opening can be allowed to be opened only while the operating knob is being manipulated, so that a function of hermetically closing the strip cartridge can be reliably maintained. A test strip can be prevented from degeneration even when used for an extended period of time, thereby improving the accuracy of a glucose measurement result.

### Technical Solution

In accordance with an aspect of the present invention, there is provided an integrated blood glucose meter as defined by claim 1. Further advantageous embodiments of the present invention are defined by the dependent claims.

The present disclosure provides an integrated blood glucose meter including: a container configured to replaceably accommodate a strip cartridge, in which a plurality of test strips are stored, in an internal space of the container, and including a strip outlet through which a test strip is discharged; a strip loading unit disposed in the container to be linearly movable in response to a manipulation of a user, wherein the strip loading unit discharges a portion of a test strip, supplied through a supply opening of the strip cartridge, to the strip outlet to be loaded in a glucose measurement mode; and a cartridge opening/closing module disposed within the container to work in concert with an operation of the strip loading unit to open and close the supply opening of the strip cartridge.

In the integrated blood glucose meter, the cartridge opening/closing module includes: a rotatable sleeve rotating and moving in concert with a linear movement of the strip loading unit; and an opening/closing cover being pressed or released from a pressed state by a rotating movement of the rotatable sleeve to open and close the supply opening of the strip cartridge. A support shaft is fitted into an internal space of the rotatable sleeve in direction of a central axis, and the rotatable sleeve is coupled to an outer circumferential surface of the support shaft so that the rotatable sleeve is rotatable around the central axis.

In addition, a pressing protrusion may protrude from an outer circumferential surface of the rotatable sleeve to press the opening/closing cover, the opening/closing cover may be mounted to be linearly movable in a top-bottom direction to open and close the supply opening of the strip cartridge, and may be elastically supported upward in a direction of contacting the rotatable sleeve by an elastic spring.

In addition, the strip loading unit may include: an operating knob connected to the container to be exposed on one surface of the container and to be linearly movable, such that the operating knob is operated by the user; a movable body coupled to the operating knob to move linearly, integrally with the operating knob; and a loading plate linearly movable, integrally with the operating knob, to load a test strip, supplied through the supply opening of the strip cartridge, into the strip outlet by pushing the test strip.

In addition, the rotatable sleeve may have a cam groove and the movable body may have a cam protrusion corresponding to the cam groove, the cam groove and the cam protrusion engaging with each other to provide a cam structure. The rotatable sleeve may rotate and move via the cam structure in response to a linear movement of the movable body.

In addition, in a state in which the cartridge opening/closing module has opened the supply opening of the strip cartridge, the loading plate may move linearly to push a test strip, thereby closing the supply opening of the strip cartridge.

In addition, a movement restriction guide guiding the strip loading unit may be provided in the container. The movement restriction guide may prevent the strip loading unit from returning to a reference position before a linear movement process of loading a test strip is completed.

In addition, the movement restriction guide may include: a movement path guide rail guiding the strip loading unit along a linear movement path for loading of a test strip; and a return path guide rail guiding the strip loading unit along a return movement path. The movement path guide rail may have a plurality of ratchet teeth to prevent the strip loading unit from returning to an initial position. The strip loading unit may have a guide protrusion to be inserted into and guided by the movement path guide rail and the return path guide rail.

In addition, a loading locking device may be provided in and the movable body may have a cam protrusion corresponding to the cam groove, the cam groove and the cam protrusion engaging with each other to provide a cam structure. The rotatable sleeve may rotate and move via the cam structure in response to a linear movement of the movable body.

In addition, in a state in which the cartridge opening/closing module has opened the supply opening of the strip cartridge, the loading plate may move linearly to push a test strip, thereby closing the supply opening of the strip cartridge.

In addition, a movement restriction guide guiding the strip loading unit may be provided in the container. The movement restriction guide may prevent the strip loading unit from returning to a reference position before a linear movement process of loading a test strip is completed.

In addition, the movement restriction guide may include: a movement path guide rail guiding the strip loading unit along a linear movement path for loading of a test strip; and a return path guide rail guiding the strip loading unit along a return movement path. The movement path guide rail may have a plurality of ratchet teeth to prevent the strip loading unit from returning to an initial position. The strip loading unit may have a guide protrusion to be inserted into and guided by the movement path guide rail and the return path guide rail.

In addition, a loading locking device may be provided in the container, the loading locking device preventing an operation of the strip loading unit in a state in which the strip cartridge is not accommodated in the container.

In addition, the loading locking device may include: a locking block disposed within the container to be linearly movable and, in response to an insertion of the strip cartridge, is engaged with the strip cartridge to move linearly in a direction in which the strip cartridge is inserted; and an elastic member applying elastic force to the locking block so that the locking block elastically moves in a direction opposite to the direction in which the strip cartridge is inserted. An operation of the strip loading unit may be blocked or unblocked, depending on a linear movement of the locking block.

In addition, the integrated blood glucose meter may further include an ejecting unit disposed in the container to be operable by a pressing manipulation of the user to discharge and remove a test strip, loaded by the strip loading unit, from the strip outlet.

In addition, the container may be provided with a lancet connector to which a lancet device is detachably attached.

### Advantageous Effects

According to the present disclosure, the strip cartridge, in which a plurality of test strips are stored, can be accommodated in the integrated blood glucose meter. Test strips can be loaded individually from the strip cartridge by a linear movement of an operating knob, so that a simple operating method and structure can be provided, and test strips can be accurately supplied and discharged.

In addition, the supply opening of the strip cartridge can be opened only while the operating knob is being manipulated, so that a function of hermetically closing the strip cartridge can be reliably maintained. A test strip can be prevented from degeneration even when used for an extended period of time, thereby improving the accuracy of a glucose measurement result.

In addition, the supply opening of the strip cartridge can be opened and closed in concert with the linear movement of the operating knob. Accordingly, the structure can be simplified, the number of components can be reduced, the manipulation can be performed easily, and the operation can be accurate.

In addition, the operating knob can be prevented from returning to the initial position during the linear movement of the operating knob. Accordingly, it is possible to discharge test strips regularly while preventing damage to test strips. In addition, it is possible to correctly determine the number of test strips remaining in the strip cartridge.

In addition, when the strip cartridge has not been inserted into the container, the operating knob cannot be manipulated. Accordingly, it is possible to excessively move or manipulate the operating knob, thereby preventing damage to the operating knob and maintaining stability.

In addition, test strips can be discharged and removed using the separate ejecting unit operable in response to a pressing manipulation. Accordingly, it is possible to conveniently perform the operation of discharging and removing test strips and prevent entrance of an external test strip or a foreign material, thereby preventing damage to an internal structure and maintaining a stabilized operating state.

### DESCRIPTION OF DRAWINGS

FIG. 1 is a perspective view schematically illustrating an external shape of an integrated blood glucose meter according to an embodiment of the present disclosure;
FIG. 2 is a perspective view schematically illustrating an internal assembly structure of the integrated blood glucose meter according to the embodiment of the present disclosure;
FIG. 3 is an exploded perspective view schematically illustrating a configuration of the integrated blood glucose meter according to the embodiment of the present disclosure;
FIG. 4 is a cross-sectional view schematically illustrating an internal structure of the integrated blood glucose meter according to the embodiment of the present disclosure;
FIG. 5 is an exploded perspective view schematically illustrating an assembly structure of a strip loading unit and a cartridge opening and closing module of the integrated blood glucose meter according to the embodiment of the present disclosure;
FIG. 6 is an operating state diagram schematically illustrating operating states of the strip loading unit and the cartridge opening and closing module of the integrated blood glucose meter according to the embodiment of the present disclosure;
FIG. 7 is a view schematically illustrating a configuration of a movement restriction guide of the integrated blood glucose meter according to the embodiment of the present disclosure;
FIG. 8 is a view schematically illustrating a configuration and an operating state of a loading operation locking device of the integrated blood glucose meter according to the embodiment of the present disclosure;
FIG. 9 is a view schematically illustrating an initial operating state of the strip loading unit of the integrated blood glucose meter according to the embodiment of the present disclosure; and
FIG. 10 is a view schematically illustrating a final operation completed state of the strip loading unit of the integrated blood glucose meter according to the embodiment of the present disclosure;
FIG. 11 is a perspective view schematically illustrating a configuration of the ejecting unit of the integrated blood glucose meter according to the embodiment of the present disclosure;
FIG. 12 is an exploded perspective view schematically illustrating specific components of the ejecting unit of the integrated blood glucose meter according to the embodiment of the present disclosure; and
FIG. 13 is a cross-sectional view schematically illustrating an internal structure and an operating state of the ejecting unit of the integrated blood glucose meter according to the embodiment of the present disclosure.

### MODE FOR INVENTION

Hereinafter, embodiments of the present disclosure will be described in detail with reference to the accompanying drawings. Throughout this document, reference should be made to the drawings, in which the same reference numerals and symbols will be used to designate the same or like components. In the following description of the present disclosure, detailed descriptions of known functions and components incorporated herein will be omitted in the case that the subject matter of the present disclosure may be rendered unclear thereby.

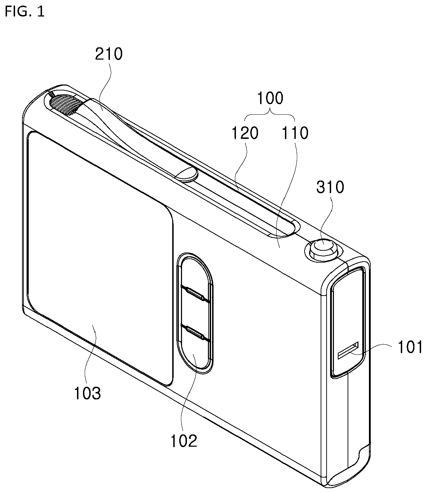

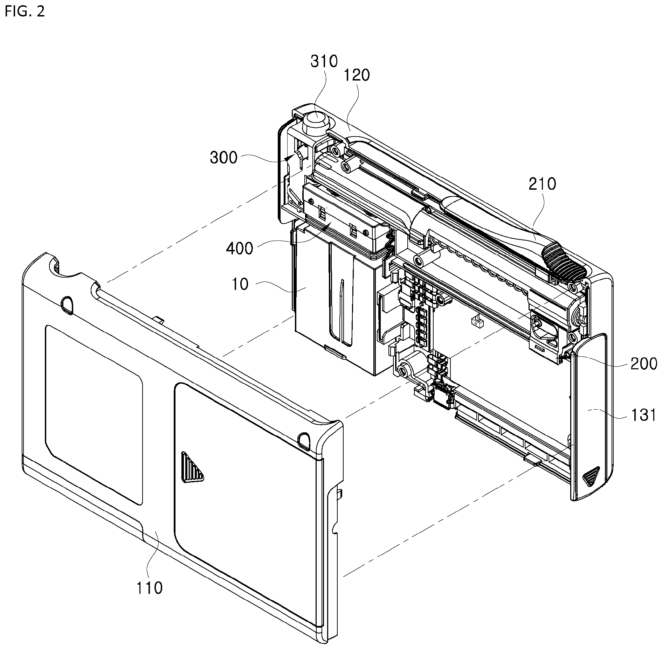

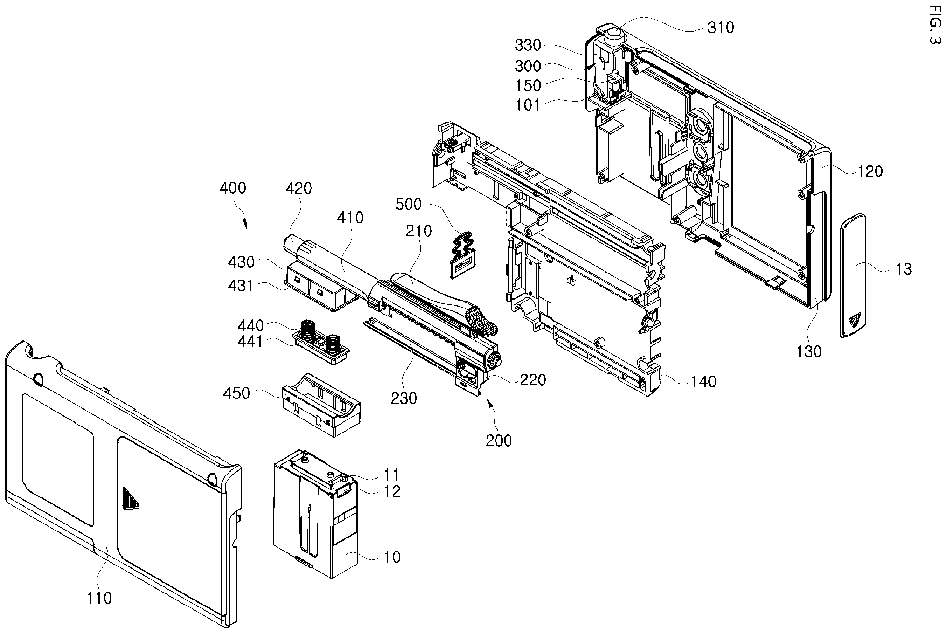

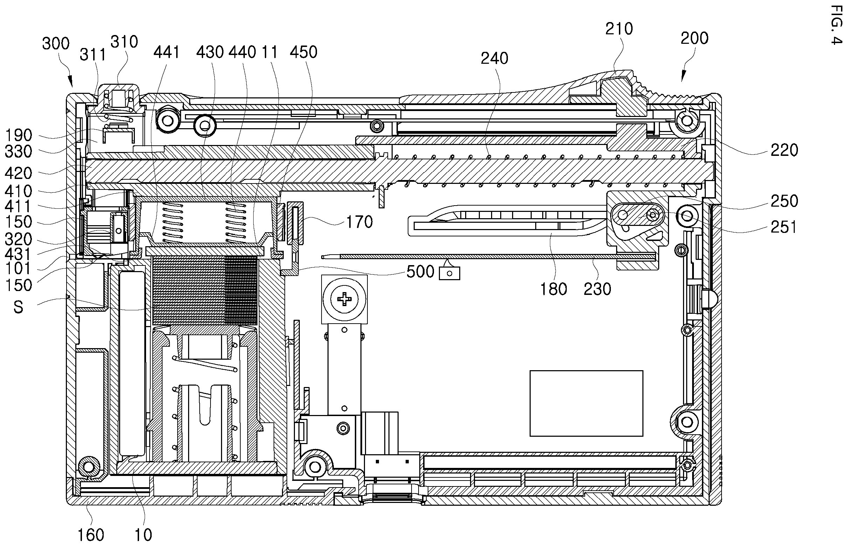

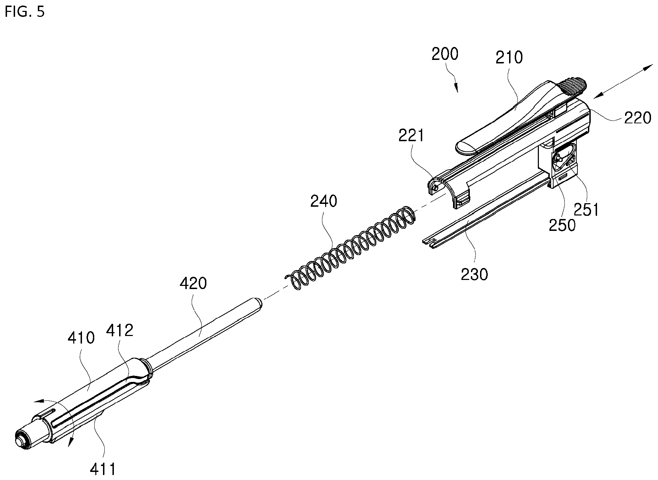

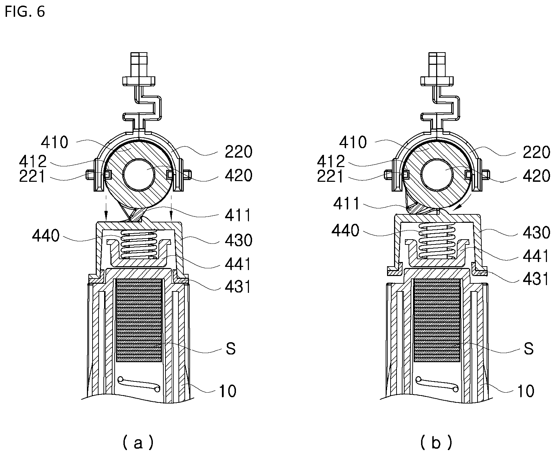

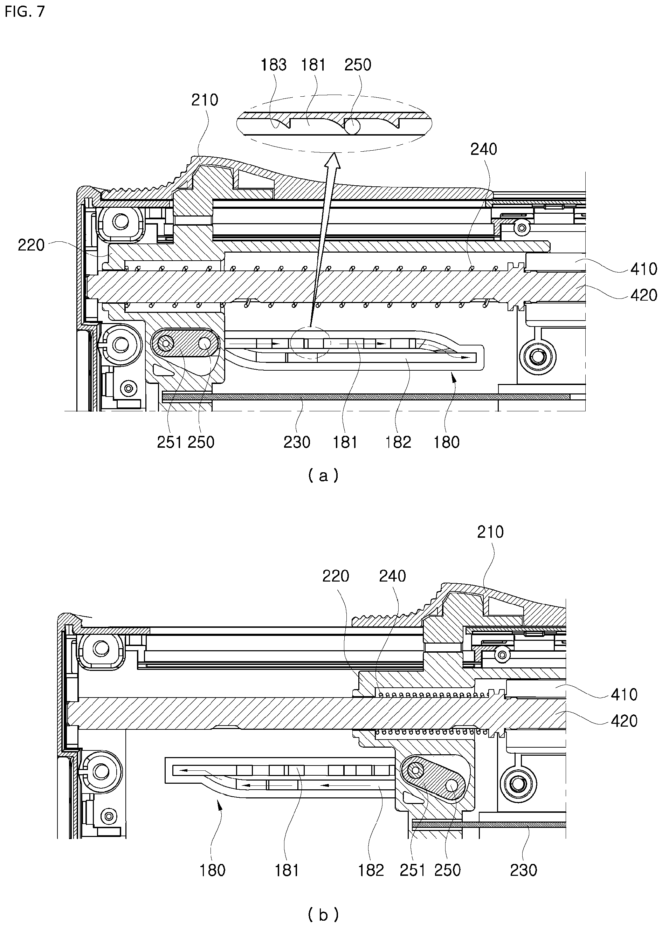

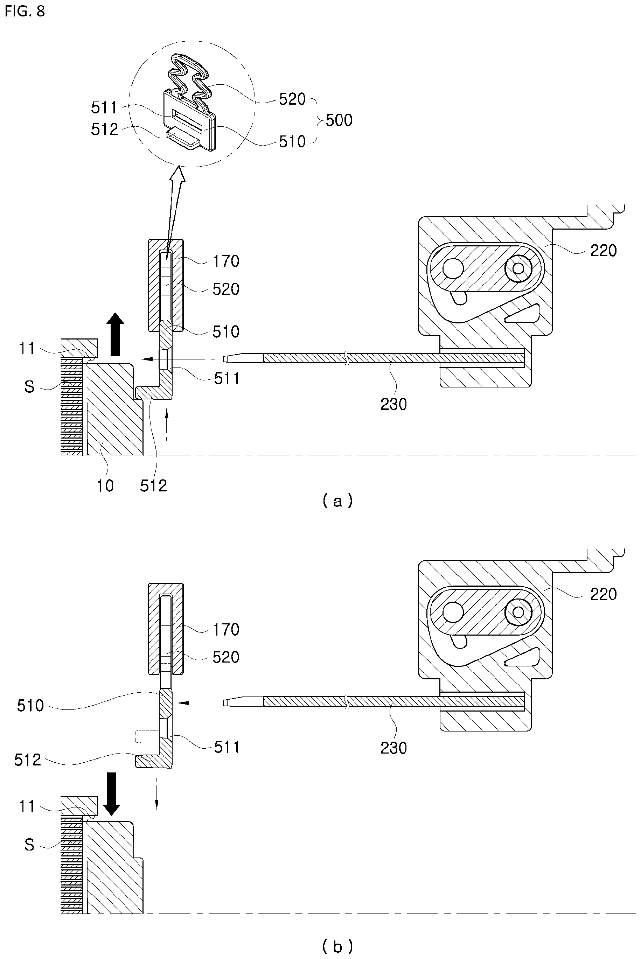

FIG. 1 is a perspective view schematically illustrating an external shape of an integrated blood glucose meter according to an embodiment of the present disclosure, FIG. 2 is a perspective view schematically illustrating an internal assembly structure of the integrated blood glucose meter according to the embodiment of the present disclosure, FIG. 3 is an exploded perspective view schematically illustrating a configuration of the integrated blood glucose meter according to the embodiment of the present disclosure, FIG. 4 is a cross-sectional view schematically illustrating an internal structure of the integrated blood glucose meter according to the embodiment of the present disclosure, FIG. 5 is an exploded perspective view schematically illustrating an assembly structure of a strip loading unit and a cartridge opening and closing module of the integrated blood glucose meter according to the embodiment of the present disclosure, FIG. 6 is an operating state diagram schematically illustrating operating states of the strip loading unit and the cartridge opening and closing module of the integrated blood glucose meter according to the embodiment of the present disclosure, FIG. 7 is a view schematically illustrating a configuration of a movement restriction guide of the integrated blood glucose meter according to the embodiment of the present disclosure, and FIG. 8 is a view schematically illustrating a configuration and an operating state of a loading operation locking device of the integrated blood glucose meter according to the embodiment of the present disclosure.

The integrated blood glucose meter according to the embodiment of the present disclosure is an integrated blood glucose meter able to accommodate a strip cartridge, in which a plurality of test strips are stored, and supply test strips individually. The integrated blood glucose meter includes a container 100, a strip loading unit 200, a cartridge opening/closing module 400, and an ejecting unit 300.

The container 100 is a component providing a contour of an entire device. The container 100 may include separate pieces, including a container body 110 and a container cover 120. A separate intermediate support plate 140 may be disposed between the container body 110 and the container cover 120, such that a plurality of components can be mounted on the intermediate support plate 140. The container 100 is configured such that the strip cartridge 10 can be replaceably accommodated in the internal space thereof and, in this regard, may be provided with a separate cartridge door 160 for opening and closing the internal space of the container 100. In addition, a control 102 and a display 103 are provided in the front portion of the container 100. The control 102 includes buttons that can be manipulated by a user. The display 103 can output and display a glucose measurement result and various pieces of information thereon.

In addition, the container 100 has a strip outlet 101 in one end portion. The strip outlet 101 allows a portion of a test strip S to be discharged from the strip cartridge 10 therethrough so as to be loaded in a blood glucose measurement mode. In addition, a connector housing 150 is disposed in a portion adjacent to the strip outlet 101, and has a connector terminal 151 mounted thereon. The connector terminal 151 can be electrically connected to a strip electrode S1 of the test strip S loaded in the strip outlet 101. In a state in which the test strip S is loaded into the strip outlet 101, the strip electrode S1 of the test strip S is in contact with and electrically connected to the connector terminal 151 of the connector housing 150. In this state, a blood glucose measuring operation is performed by causing a blood sample to be absorbed by the test strip S. After the blood glucose measuring operation is completed, the used test strip S is discarded after being completely discharged from the container 100. The separate ejecting unit 300 may be provided to perform the operation of discharging the test strip S.

In addition, the container 100 is provided with a lancet connector 130, to which a lancet device (not shown) can be detachably attached. The lancet connector 130 may be configured such that the lancet device (not shown) can be connected thereto by sliding. In a case in which the lancet device is not provided, a finishing plate 131 may be provided to be connected to the lancet connector 130. The lancet device may have a variety of configurations respectively provided with an internal lancet, such that blood can be collected using the lancet.

The strip cartridge 10 is configured such that a plurality of test strips S are stacked vertically on each other (or stacked on each other in the top-bottom direction). The strip cartridge 10 has a supply opening 11 in the upper portion and an elastic spring disposed in the lower portion, such that stacked test strips S are pushed upwardly by the elastic force of the elastic spring. Accordingly, when one test strip S is ejected outwardly through the supply opening 11, another test strip S stacked therebelow is moved upwardly toward the supply opening 11 so as to be ready to be supplied.

In addition, test strips S may be accommodated in a sealed state, since test strips S are susceptible to being degraded by external moisture. Although not shown, a separate piece of sealing tape (not shown) may be attached to the supply opening 11. Test strips S may be configured to be inserted into container 100 at the moment at which the sealing tape is removed. In addition, it is important to maintain the sealing state for the internal space of the strip cartridge 10 after insertion into the container 100. This can be performed by the cartridge opening/closing module 400.

The strip cartridge 10 is configured such that the strip cartridge 10 can be replaced with a new strip cartridge after all of test strips S stored therein have been used. The strip cartridge 10 may be configured to be easily attached to and detached from the container 100 using a separate fixing clip (not shown) or the like.

The strip loading unit 200 is disposed in the container 100 so as to be linearly movable in response to a manipulation of the user. A portion of each test strip S, supplied through the supply opening 11 of the strip cartridge 10, is discharged to the strip outlet 101 to be loaded in the blood glucose measurement mode.

The cartridge opening/closing module 400 is disposed within the container 100, and works in concert with the operation of the strip loading unit 200 to open and close the supply opening 11 of the strip cartridge 10.

That is, in a reference state in which the strip loading unit 200 has not been loaded, the cartridge opening/closing module 400 is operated to close the supply opening 11 of the strip cartridge 10. When the strip loading unit 200 is operated to load the test strip S from the strip cartridge 10, the cartridge opening/closing module 400 is operated to open the supply opening 11 of the strip cartridge 10. Since the cartridge opening/closing module 400 operates in concert with the operation of the strip loading unit 200, the supply opening 11 of the strip cartridge 10 is opened only in the process of loading the test strip S by the strip loading unit 200. When the process of loading the test strip S is completed, the strip loading unit 200 returns to the reference position, so that the supply opening 11 of the strip cartridge 10 is closed by the cartridge opening/closing module 400.

The ejecting unit 300 operates to discharge and remove the test strip S, loaded into the strip outlet 101 by the strip loading unit 200, from the strip outlet 101. The ejecting unit 300 is mounted on the container 100 such that the user can operate the ejecting unit 300 by pressing the ejecting unit 300.

According to this structure, it is possible to load the test strip S individually into the strip outlet 101 of the strip cartridge 10 from by manipulating the strip loading unit 200 to move linearly. In a state in which a test strip S is loaded, the blood glucose measuring operation is performed. When the blood glucose measuring operation is completed, the test strip S can be discharged and removed from the strip outlet 101 by manipulating the ejecting unit 300.

Next, specific configurations of the integrated blood glucose meter according to the embodiment of the present disclosure will be described in more detail.

The strip loading unit 200 includes an operating knob 210, a movable body 220, and a loading plate 230. The operating knob 210 is connected to the container 100 to be exposed on one surface of the container 100 and to be linearly movable, such that the operating knob 210 can be operated by the user. The movable body 220 is coupled to the operating knob 210 to move linearly, integrally with the operating knob 210. The loading plate 230 is linearly movable, integrally with the operating knob 210, to load the test strip S, supplied through the supply opening 11 of the strip cartridge 10, into the strip outlet 101 by pushing the test strip S. In addition, the strip loading unit 200 is configured to be restored to the initial reference position by a separate elastic spring 240. Accordingly, after the strip loading unit 200 has been moved linearly, when the user removes the manipulating force, the strip loading unit 200 is automatically restored to the initial reference position by the elastic force of the elastic spring 240.

The cartridge opening/closing module 400 includes a rotatable sleeve 410 and an opening/closing cover 430. The rotatable sleeve 410 rotates and moves in concert with a linear movement of the strip loading unit 200. The opening/closing cover 430 is pressed or released from a pressed state by the rotating movement of the rotatable sleeve 410 to open and close the supply opening 11 of the strip cartridge 10. Here, a pressing protrusion 411 protrudes from an outer circumferential surface of the rotatable sleeve 410, such that the opening/closing cover 430 can be pressed by the pressing protrusion 411. The opening/closing cover 430 is mounted to be linearly movable in the top-bottom direction to open and close the supply opening 11 of the strip cartridge 10, and is elastically supported upward in a direction of contacting the rotatable sleeve 410 by a separate elastic spring 440.

The opening/closing cover 430 may have the shape of a vessel, with the bottom end surface thereof being open. A sealing member 431 may be mounted on a lower peripheral portion of the opening/closing cover 430 to close the supply opening 11 of the strip cartridge 10 in a sealing manner. The elastic spring 440 is supported by a separate support plate 441 to elastically support the opening/closing cover 430. A separate base housing 450 may be disposed outside of the opening/closing cover 430 to guide the opening/closing cover 430 along a vertical linear movement path (or a linear movement path in a top-bottom direction).

A separate support shaft 420 is fitted into an internal space of the rotatable sleeve 410. The rotatable sleeve 410 is coupled to the outer circumferential surface of the separate support shaft 420 to be rotatable and movable.

Here, the structure in which the rotatable sleeve 410 rotates and moves in concert with the linear movement of the strip loading unit 200 can be realized by a structure having a cam structure in which the movable body 220 of the strip loading unit 200 is engaged with the rotatable sleeve 410. For example, as illustrated in FIG. 5, a cam groove 412 having an initial curved path portion may be provided in the outer circumferential surface of the rotatable sleeve 410. A cam protrusion 221 engaging with the cam groove 412 may be provided in an inner circumferential portion of the movable body 220.

According to this structure, when the movable body 220 of the strip loading unit 200 moves linearly, the cam protrusion 221 and the cam groove 412 move together while being engaged with each other. Due to the engagement structure of the cam protrusion 221 and the cam groove 412, the rotatable sleeve 410 rotates and moves around the support shaft 420. Here, the rotatable sleeve 410 rotates in the process in which the movable body 220 moves linearly and the cam protrusion 221 moves in engagement with the initial curved path portion, and thereafter, the cam groove 421 moves linearly along a linear movement path portion. Accordingly, the rotatable sleeve 410 maintains the initial state of rotation.

As illustrated in FIG. 6 (a), the pressing protrusion 411 of the rotatable sleeve 410 is configured to press the opening/closing cover 430 in the reference state in which the strip loading unit 200 does not operate. When the rotatable sleeve 410 rotates and moves in response to the operation of the strip loading unit 200, the pressing protrusion 411 provided on the outer circumferential surface of the rotatable sleeve 410 rotates, as illustrated in FIG. 6 (b). Accordingly, the opening/closing cover 430 of the pressing protrusion 411 is released from being pressed, so that the opening/closing cover 430 is moved upwardly by the elastic force of the elastic spring 440 to open the supply opening 11 of the strip cartridge 10. At the same time, the loading plate 230 of the strip loading unit 200 pushes the test strip S, exposed by the supply opening 11 of the strip cartridge 10, while being moved linearly, so that the test strip S is discharged through the strip outlet 101. Here, the loading plate 230 is configured to close the supply opening 11 of the strip cartridge 10 in the process of pushing the test strip S.

According to this structure, the cartridge opening/closing module 400 is maintained to constantly close the supply opening 11 of the strip cartridge 10 in a sealing manner, pressed by the pressing protrusion 411 of the rotatable sleeve 410. Only in a case in which the user operates the strip loading unit 200 to move linearly, the opening/closing cover 430 is released from the state of being pressed by the pressing protrusion 411 of the rotatable sleeve 410. After the linear movement of the strip loading unit 200 is completed, the strip loading unit 200 is restored to the reference state by the elastic spring 240. Here, since the rotatable sleeve 410 is rotated and restored to the initial position so that the opening/closing cover 430 is pressed by the pressing protrusion 411, the supply opening 11 of the strip cartridge 10 is closed in a sealing manner. In particular, since the supply opening 11 is temporarily closed by the loading plate 230 even in a state in which the strip loading unit 200 is moved linearly, the sealing state of the internal space of the strip cartridge 10 can be more reliably maintained.

In addition, a movement restriction guide 180 guiding the strip loading unit 200 is provided in the container 100. The movement restriction guide 180 prevents the strip loading unit 200 from returning to the reference position before the linear movement process of loading the test strip S is completed.

As illustrated in FIG. 7, a separate guide protrusion 250 protrudes from a portion of the movable body 220 of the strip loading unit 200. The guide protrusion 250 may be coupled to a guide block 251 pivotably coupled to the movable body 220, such that the guide protrusion 250 is rotatable and movable in a specific path portion. The movement restriction guide 180 guides the strip loading unit 200 along a movement path, while restraining the strip loading unit 200 returning to the initial position, by engagement with the guide protrusion 250.

The movement restriction guide 180 includes a movement path guide rail 181 and a return path guide rail 182, as illustrated in FIG. 7. The movement path guide rail 181 guides the strip loading unit 200 along a linear movement path for the loading of the test strip S. The return path guide rail 182 guides the strip loading unit 200 along a return movement path. Here, the movement path guide rail 181 has a plurality of ratchet teeth 183 provided on intermediate portions thereof to prevent the strip loading unit 200 from returning to the initial position. The strip loading unit 200 is configured such that the guide protrusion 250 provided on the movable body 220 is inserted into and moves while being guided by the movement path guide rail 181 and the return path guide rail 182.

Accordingly, in the strip loading unit 200, as illustrated in FIG. 7 (a), the guide protrusion 250 moves on the movement path guide rail 181 along the linear movement path portion for the loading of the test strip S. After the loading linear movement for loading the test strip S has been completed, the guide protrusion 250 returns to the initial position along the return path guide rail 182, as illustrated in FIG. 7 (b) . Here, in the case of moving the guide protrusion 250 linearly along the loading linear movement path portion for loading the test strip S, the guide protrusion 250 moves smoothly without engaging with the guide protrusion 250. In contrast, when it is attempted to return the guide protrusion 250 to the initial position during the linear movement in this path portion, i.e. the linear movement path portion, the guide protrusion 250 cannot return to the initial position due to engagement with the ratchet teeth 183, since the ratchet teeth 183 are provided on the movement path guide rail 181. Accordingly, when the operating knob 210 is initially moved linearly for the loading, the operating knob 210 cannot return to the initial position until the loading linear movement is completed.

That is, even in the case in which the user accidentally or intentionally releases the operating knob 210 while linearly moving the operating knob 210 for the loading, the operating knob 210 and the strip loading unit 200, including the strip loading unit 200, are fixed in a position in which the operating knob 210 is released, instead of returning to the reference position.

This structure can prevent the discharging of the test strip S from being stopped in the intermediate step of discharging the test strip S. In this manner, a range of additional functions of allowing test strips S to be discharged regularly, preventing test strips S from being damaged, and managing the number of test strip S remaining in the strip cartridge 10 can be achieved.

In addition, a loading locking device 500 may be provided in the container 100. The loading locking device 500 prevents the operation of the strip loading unit 200 in a state in which the strip cartridge 10 is not accommodated in the container 100.

The loading locking device 500 may include a locking block 510 and an elastic member 520. The locking block 510 is disposed within the container 100 to be linearly movable and, in response to the insertion of the strip cartridge 10, is engaged with the strip cartridge 10 to move linearly in a direction in which the strip cartridge 10 is inserted. The elastic member 520 applies elastic force to the locking block 510 so that the locking block 510 elastically moves in the direction opposite to the direction in which the strip cartridge 10 is inserted. The loading locking device 500 may be configured to block the operation, or unblock the blocked operation, of the strip loading unit 200, depending on the linear movement of the locking block 510. The elastic member 52 may be formed of an elastic material, such as rubber, having a shape, such as a zigzag shape, by which elastic force can be exerted. Alternatively, the elastic member may be implemented as a typical elastic spring.

In addition, the container 100 may have a locking guide 170 guiding paths of operation of the locking block 510 and the elastic member 52, while the strip cartridge 10 may have a stepped recess 12 engaging with the locking block 510. In addition, the locking block 510 may have a fitting protrusion 512 engaging with the stepped recess 12 of the strip cartridge 10. A passage hole 511 may be provided in a central area, allowing the loading plate 230 of the strip loading unit 200 to pass through the passage hole 511.

According to this structure, when the strip cartridge 10 is inserted into the container 100, the fitting protrusion 512 of the locking block 510 engages with the stepped recess 12 of the strip cartridge 10, so that the locking block 510 moves upwardly in the direction in which the strip cartridge 10 is inserted, as illustrated in FIG. 8 (a). Then, the elastic member 52 is elastically pressed. In response to the locking block 510 moving upwardly as described above, the position of the passage hole 511 of the locking block 510 is moved upwardly. In this state, the position of the passage hole 511 is located on the path for the loading linear movement of the loading plate 230 of the strip loading unit. Accordingly, in this state, when the strip loading unit 200 is moved by manipulation, the loading plate 230 can move linearly through the passage hole 511, so that the strip loading unit 200 can operate properly.

In contrast, when the strip cartridge 10 is removed from the container 100, the stepped recess 12 of the strip cartridge 10 is disengaged from the fitting protrusion 512 of the locking block 510, so that the locking block 510 is moved downwardly by the elastic force of the elastic member 52, as illustrated in FIG. 8 (b). At this time, the position of the passage hole 511 of the locking block 510 is also moved downwardly. Consequently, in this state, the linear movement of the loading plate 230 is blocked by the locking block 510. That is, when the strip cartridge 10 is removed from the container 100, the linear movement of the loading plate 230 is blocked by the locking block 510, so that the operation of the strip loading unit 200 is disabled.

According to this structure, in a state in which the strip cartridge 10 is not inserted through the loading locking device 500, the integrated blood glucose meter according to the embodiment of the present disclosure can prevent the operating knob 210 from operating, thereby preventing the operating knob 210 and the strip loading unit 200 from moving excessively by manipulation and maintaining stability.

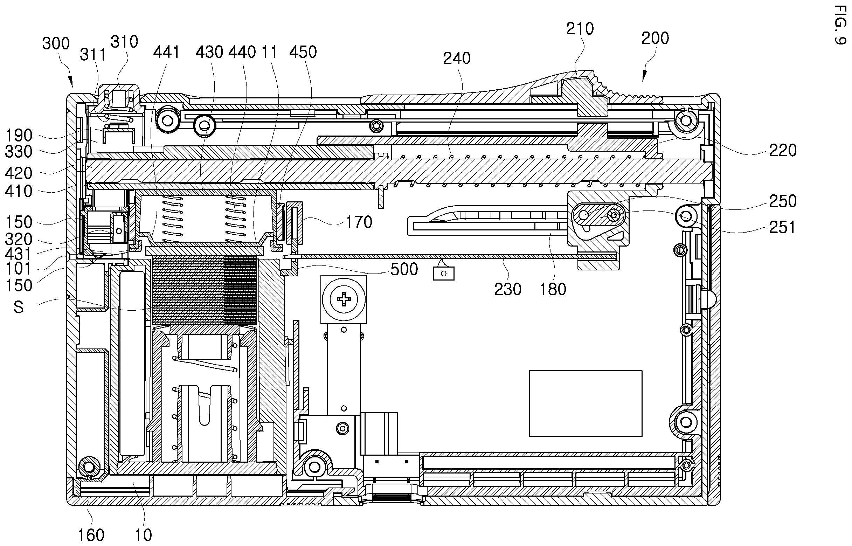

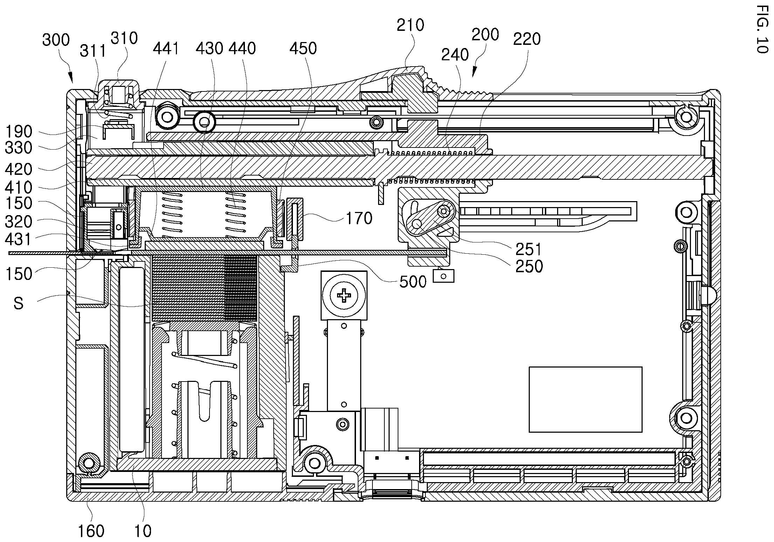

The operation of the integrated blood glucose meter according to the embodiment of the present disclosure, having the above-described structure, will be described with reference to FIGS. 9 and 10.

FIG. 9 is a view schematically illustrating an initial operating state of the strip loading unit of the integrated blood glucose meter according to the embodiment of the present disclosure, while FIG. 10 is a view schematically illustrating a final operation completed state of the strip loading unit of the integrated blood glucose meter according to the embodiment of the present disclosure.

FIG. 4 illustrates a state in which the strip loading unit 200 is located in the reference position, while FIG. 9 illustrates a state in which the strip loading unit 200 has been initially moved. As illustrated in FIG. 9, when the operating knob 210 starts the loading linear movement to the left with respect to the illustration direction, the movable body 220 moves along with the operating knob 210, and the rotatable sleeve 410 connected to the movable body 220 via the cam structure rotates. At the same time, the pressing protrusion 411 of the rotatable sleeve 410 rotates, so that the opening/closing cover 430 is released from the state of being pressed by the pressing protrusion 411. Then, the opening/closing cover 430 is moved upwardly by the elastic force of the elastic spring 440, thereby opening the supply opening 11 of the strip cartridge 10. Here, the loading plate 230 is located adjacent to the supply opening 11.

Afterwards, when the operating knob 210 is further moved linearly to the left, the operating knob 210 moves linearly to an end point of the loading linear movement path, as illustrated in FIG. 10. In this process, the loading plate 230 moves to push a test strip S from the supply opening 11 of the strip cartridge 10 through the strip outlet 101. Then, a portion of the test strip S is discharged to and loaded in the strip outlet 101, so as to remain in contact with the connector terminal 151 of the connector housing 150 mounted around the strip outlet 101. In this state, the user can perform a blood glucose measuring operation by causing a blood sample to be absorbed by the discharged portion of the loaded test strip S.

Since the loading plate 230 closes the supply opening 11 of the strip cartridge 10 in the state in which the test strip S is loaded as described above, the sealed state of the internal space of the strip cartridge 10 is maintained in this state. Afterwards, when the manipulating force applied to the operating knob 210 is removed, the strip loading unit 200 returns to the reference position due to the elastic force of the elastic spring 240. In the returning process, the rotatable sleeve 410 is rotated in the opposite direction to press the opening/closing cover 430 via the pressing protrusion 411, thereby closing the supply opening 11 of the strip cartridge 10.

The test strip S is loaded from the strip cartridge 10 to the strip outlet 101 as described above. In this state, the blood glucose measuring operation is performed by causing the blood sample to be absorbed by the test strip S. When the blood glucose measuring operation is completed, it is possible to completely discharge and remove the test strip S from the strip outlet 101 in which the test strip S has been loaded by performing a pressing manipulation to the ejecting unit 300.

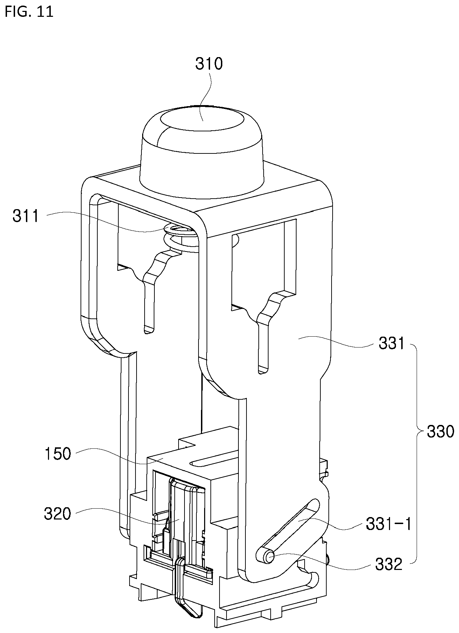

Next, a configuration of the ejecting unit according to the embodiment of the present disclosure will be described in more detail with reference to FIGS. 11 to 13.

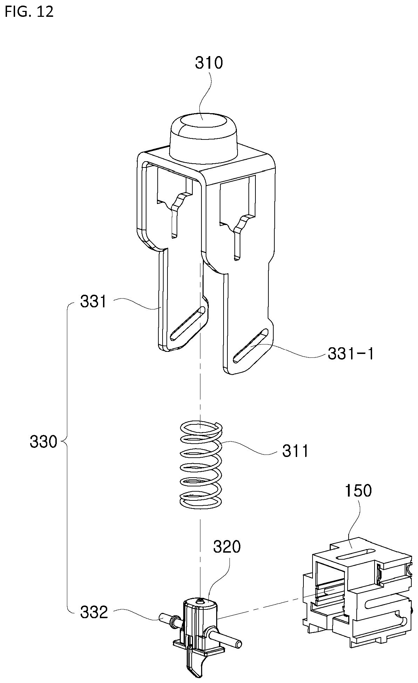

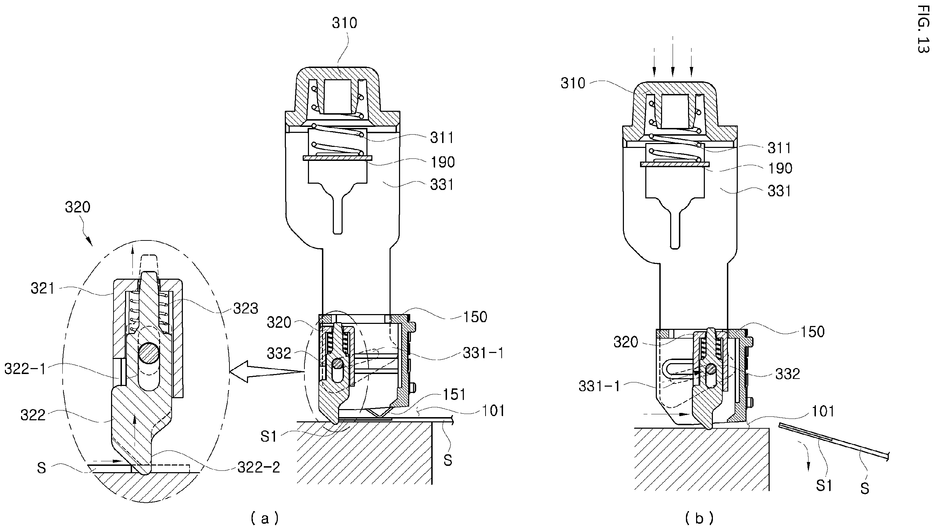

FIG. 11 is a perspective view schematically illustrating a configuration of the ejecting unit of the integrated blood glucose meter according to the embodiment of the present disclosure, FIG. 12 is an exploded perspective view schematically illustrating specific components of the ejecting unit of the integrated blood glucose meter according to the embodiment of the present disclosure, and FIG. 13 is a cross-sectional view schematically illustrating an internal structure and an operating state of the ejecting unit of the integrated blood glucose meter according to the embodiment of the present disclosure.

The ejecting unit 300 is disposed in a position adjacent to the strip outlet 101 of the container 100, and is configured to be operable in response to a pressing manipulation of the user to discharge and remove the test strip S from the strip outlet 101 in which the test strip S has been loaded.

The above-described ejecting unit 300 is configured to convert unidirectional moving force, generated by the pressing manipulation of the user, into perpendicular moving force, by which the test strip S is discharged.

After the blood glucose measuring operation is completed by the ejecting unit 300, it is possible to conveniently and simply discharge and remove the test strip S from the strip outlet 101 by a simple pressing manipulation. In particular, the test strip S is discharged by a mechanism converting the pressing manipulating force of the user into perpendicular moving force. Here, the test strip is discharged by simply converting the manipulating force of the user into force acting in a direction perpendicular to the manipulating force, instead of discharging the test strip by separate elastic force. Thus, when the test strip S is discharged, the test strip S is discharged at an adequate speed by the manipulating force of the user, instead of being ejected too forcefully. Accordingly, the discharged test strip S can be easily input to a garbage can, and thus the user can conveniently discharge and remove the test strip S without having to touch the test strip S with a hand.

Describing the configuration of the ejecting unit 300 in more detail, the ejecting unit 300 includes a press button 310, a discharge block module 320, and a linking means 330. The press button 310 is coupled to the container 100 to be movable vertically (or movable in the top-bottom direction), such that the press button 310 can be operated by a pressing manipulation of the user, and is elastically and upwardly supported by a separate elastic spring 311. The discharge block module 320 is coupled to the connector housing 150 of the strip outlet 101 to be movable horizontally, such that the discharge block module 320 moves horizontally in concert with the vertical movement of the press button 310. The linking means 330 allows the press button 310 and the discharge block module 320 to work in concert with each other. The test strip S is discharged by the horizontal movement of the discharge block module 320 working in concert with the press button 310. Here, the press button 310 is elastically and upwardly supported by the elastic spring 311. The container 100 may be provided with a separate button support 190 to support the elastic spring 311.

Here, the linking means 330 includes a guide plate 331 and a guide rod 332. The guide plate 331 is coupled to the press button 310 to move vertically, integrally with the press button 310, and has inclined guide slits 331-1. The guide rod 332 is coupled to the discharge block module 320 to move integrally with the discharge block module 320. The guide rod 332 moves along the inclined guide slits 331-1, with end portions of the guide rod 332 being inserted into the inclined guide slits 331-1.

The guide plate 331 and the guide rod 332 of the linking means 330 may be provided integrally on the press button 310 and the discharge block module 320, respectively, although guide plate 331 and the guide rod 332 may be detachably attached to the press button 310 and the discharge block module 320, respectively.

For example, the guide plate 331 may be fabricated integrally with the press button 310, molded from a synthetic resin. The guide rod 332 may be separately fabricated from a metal to be coupled to the discharge block module 320 using a separate fastener, such that the guide rod 332 extends through the discharge block module 320. When the guide plate 331 is formed of the synthetic resin, such as polyoxymethylene (POM) and the guide rod 332 is formed of the metal, such as stainless steel, the performance of the operation is improved due to relatively low friction between metal and synthetic resin and low friction noise.

The discharge block module 320 includes a discharge block 321, a catch body 322, and an elastic spring 323. The discharge block 321 is coupled to the connector housing 150 to be movable horizontally (or in a lateral direction). The catch body 322 is coupled to the discharge block 321 to be movable vertically, and has a catch portion 322-2 on the bottom end portion. The catch portion 322-2 can engage with the test strip S loaded in the strip outlet 101. The elastic spring 323 elastically supports the catch body 322 downwardly, such that the catch portion 322-2 of the catch body 322 protrudes downwardly from the discharge block 321. The guide rod 332 is coupled to the discharge block module 320 while extending through the discharge block module 320. The catch body 322 has a slot 322-1 provided in the central portion to extend in a top-bottom direction. The slot 322-1 allows the catch body 322 to move vertically.

In addition, in the catch portion 322-2 of the catch body 322, a side surface facing outward along the direction of the strip outlet 101 has a vertical surface (or a surface extending in a top-bottom direction) able to engage with the test strip S, and a side surface facing inward along the direction of the strip outlet 101 has an inclined surface.

The operating state of the ejecting unit 300 having the above-described structure will be described. First, in a state in which no pressing manipulation is performed on the press button 310, the press button 310 remains in an upwardly-moved position in the container 100 due to the elastic force of the elastic spring 311, as illustrated in FIG. 13 (a). Thus, the guide plate 331 moving integrally with the press button 310 is also in the upwardly-moved position. In this state, the guide rod 332, inserted into the inclined guide slits 331-1 of the guide plate 331, remains in position after having been moved to the left along the inclined guide slits 331-1 with respect to the direction illustrated in FIG. 13. The discharge block module 320, as well as the guide rod 332, remains in position after having been moved to the left. In this case, the test strip S remains in the loaded position, with a portion of the test strip S being discharged through the strip outlet 101. The test strip S has the strip electrode S1 able to transmit and receive an electrical signal in the glucose measurement process. With the test strip S being loaded in the strip outlet 101, the strip electrode S1 of the test strip S remains in contact with the connector terminal 151 of the connector housing 150. The connector terminal 151 is electrically connected to separate components (not shown), including a glucose measurement module and a control, disposed inside of the container 100.

In this state, when the press button 310 is pressed downwardly as illustrated in FIG. 13 (b), the press button 310 is moved downwardly within the container 100, and the guide plate 331 is also moved downwardly along with the press button 310. When the guide plate 331 is moved downwardly, the guide rod 332 is moved linearly along the inclined guide slits 331-1, to the right with respect to the direction illustrated in FIG. 13. The discharge block module 320 is moved to the right within the connector housing 150, along with the guide rod 332. Responsively, the catch portion 322-2 of the discharge block module 320 discharges the test strip S.

As described above, the ejecting unit 300 according to the embodiment of the present disclosure discharges the test strip S by the pressing manipulating force applied by the user, instead of discharging the test strip S by separate elastic force. Thus, in the process of discharging the test strip S, the test strip S is discharged at an adequate speed by the manipulating force of the user, instead of being ejected too forcefully. Accordingly, the discharged test strip S can be easily input to a garbage can, directly after being discharged, thereby further facilitating the use. In addition, the ejecting unit 300 can be easily fabricated and assembled, due to the simple confirmation thereof, thereby reducing fabrication costs. The size of the ejecting unit 300 can be reduced, thereby improving the space efficiency within the integrated blood glucose meter.

As described above, the ejecting unit 300 according to the embodiment of the present disclosure discharges the test strip S by horizontally moving the discharge block module 320 by performing a downward pressing manipulation to the press button 310 working in contact with the discharge block module 320. At this time, the catch portion 322-2 of the discharge block module 320 is positioned to protrude more downwardly than the strip outlet 101, such that the catch portion 322-2 of discharges the test strip S during the movement of the catch portion 322-2.

In this case, in the process of discharging and loading a portion of the test strip S by pushing the test strip S from the strip cartridge 10 using the strip loading unit 200, the movement of the test strip S may be interfered with by the catch portion 322-2 of the catch body 322, so that the loading process may not be performed properly. To prevent this, in the discharge block module 320, the catch body 322 having the catch portion 322-2 may be coupled to the discharge block 321 to be movable vertically, and one surface of the catch portion 322-2 may have the inclined surface, as described above. According to this structure, as illustrated in the enlarged view of FIG. 13 (a), in the process of loading the test strip S in the strip outlet 101, the test strip S is brought into contact with the catch portion 322-2. Then, the test strip S is moved linearly to be loaded in the strip outlet 101 without interference. At the same time, in response to the linear movement of the test strip S, the catch portion 322-2 and the catch body 322 are moved upwardly due to the inclined surface of the catch portion 322-2. When the loading movement of the test strip S is completed properly, the catch body 322 and the catch portion 322-2 are moved downwardly again by the elastic force of the elastic spring 323 so as to return to the initial position.

In addition, in the catch portion 322-2 of the catch body 322, the side surface facing outward of the strip outlet 101 is provided to have the vertical surface, as described above. This structure can reliably discharge the test strip S by pushing the test strip S outwardly in response to the movement of the catch portion 322-2. At the same time, when a further test strip S or a sheet-shaped foreign material is introduced to the strip outlet 101, the further test strip S or the sheet-shaped material can be prevented from entering the internal space of the container 100. When the further test strip S or the sheet-shaped material is introduced from the outside, neither the catch portion 322-2 nor the catch body 322 can be moved upwardly, due to the contact structure defined by the vertical side surface of the catch portion 322-2 being in contact with the further test strip S or the sheet-shaped material. Accordingly, it is possible to prevent the further test strip S or the sheet-shaped material from being introduced from the outside.

The foregoing descriptions have been presented in order to explain certain principles of the present disclosure by way of example. A person skilled in the art to which the present disclosure relates could make various modifications and variations without departing from the essential features of the present disclosure. The foregoing embodiments disclosed herein shall be interpreted as being illustrative, while not being limitative, of the principle and scope of the present disclosure. It should be understood that the scope of the present disclosure shall be defined by the appended claims.

## Claims

1. An integrated blood glucose meter comprising:
a container (100) configured to replaceably accommodate a strip cartridge (10), in which a plurality of test strips are stored, in an internal space of the container, and including a strip outlet (101) through which a test strip (S) is discharged;
a strip loading unit (200) disposed in the container to be linearly movable in response to a manipulation of a user, wherein the strip loading unit discharges a portion of a test strip, supplied through a supply opening (11) of the strip cartridge (10), to the strip outlet (101) to be loaded in a glucose measurement mode; and
a cartridge opening/closing module (400) disposed within the container to work in concert with an operation of the strip loading unit (200) to open and close the supply opening (11) of the strip cartridge (10), **characterized in that:**
the cartridge opening/closing module (400) includes:
a rotatable sleeve (410) rotating and moving in concert with a linear movement of the strip loading unit (200); and
an opening/closing cover (430) being pressed or released from a pressed state by a rotating movement of the rotatable sleeve (410) to open and close the supply opening (11) of the strip cartridge (10),
wherein a support shaft (420) is fitted into an internal space of the rotatable sleeve (410) in direction of a central axis, and the rotatable sleeve (410) is coupled to an outer circumferential surface of the support shaft (420) so that the rotatable sleeve (410) is rotatable around the central axis.

2. The integrated blood glucose meter according to claim 1, wherein a pressing protrusion (411) protrudes from an outer circumferential surface of the rotatable sleeve (410) to press the opening/closing cover (430),
the opening/closing cover (430) is mounted to be linearly movable in a top-bottom direction to open and close the supply opening (11) of the strip cartridge (10), and is elastically supported upward in a direction of contacting the rotatable sleeve (410) by an elastic spring (240).

3. The integrated blood glucose meter according to claim 1, wherein the strip loading unit (200) includes:
an operating knob (210) connected to the container (100) to be exposed on one surface of the container and to be linearly movable, such that the operating knob is operated by the user;
a movable body (220) coupled to the operating knob (210) to move linearly, integrally with the operating knob; and
a loading plate (230) linearly movable, integrally with the operating knob (210), to load a test strip (S), supplied through the supply opening (11) of the strip cartridge (10), into the strip outlet (101) by pushing the test strip.

4. The integrated blood glucose meter according to claim 3, wherein the rotatable sleeve (410) has a cam groove (412) and the movable body (220) has a cam protrusion (221) corresponding to the cam groove (412), the cam groove (412) and the cam protrusion (221) engaging with each other to provide a cam structure, and the rotatable sleeve (410) is configured to rotate and move via the cam structure in response to a linear movement of the movable body (220).

5. The integrated blood glucose meter according to claim 3, wherein, in a state in which the cartridge opening/closing module (400) has opened the supply opening (11) of the strip cartridge (10), the loading plate (230) moves linearly to push a test strip (S), thereby closing the supply opening (11) of the strip cartridge (10).

6. The integrated blood glucose meter according to claim 3, wherein a movement restriction guide (180) guiding the strip loading unit (220) is provided in the container (100), the movement restriction guide (180) preventing the strip loading unit (200) from returning to a reference position before a linear movement process of loading a test strip is completed.

7. The integrated blood glucose meter according to claim 6, wherein the movement restriction guide (180) includes:
a movement path guide rail (181) guiding the strip loading unit (200) along a linear movement path for loading of a test strip; and
a return path guide rail (182) guiding the strip loading unit (200) along a return movement path,
wherein the movement path guide rail (181) has a plurality of ratchet teeth (183) to prevent the strip loading unit (200) from returning to an initial position, and the strip loading unit has a guide protrusion (250) to be inserted into and guided by the movement path guide rail (181) and the return path guide rail (182).

8. The integrated blood glucose meter according to one of claims 1 to 7, wherein a loading locking device (500) is provided in the container (100), the loading locking device (500) preventing an operation of the strip loading unit (200) in a state in which the strip cartridge (10) is not accommodated in the container (100).

9. The integrated blood glucose meter according to claim 8, wherein the loading locking device (500) includes:
a locking block (510) disposed within the container (100) to be linearly movable and, in response to an insertion of the strip cartridge (10), is engaged with the strip cartridge to move linearly in a direction in which the strip cartridge is inserted; and
an elastic member (520) applying elastic force to the locking block (510) so that the locking block elastically moves in a direction opposite to the direction in which the strip cartridge (10) is inserted,
wherein an operation of the strip loading unit (200) is blocked or unblocked, depending on a linear movement of the locking block (510).

10. The integrated blood glucose meter according to one of claims 1 to 7, further comprising an ejecting unit (300) disposed in the container (100) to be operable by a pressing manipulation of the user to discharge and remove a test strip (S), loaded by the strip loading unit (200), from the strip outlet (101).

11. The integrated blood glucose meter according to one of claims 1 to 7, wherein the container (100) is provided with a lancet connector (130) to which a lancet device is detachably attached.

## Patentansprüche

1. Integriertes Blutzuckermessgerät, umfassend:
einen Behälter (100), der konfiguriert ist, um eine Streifenkartusche (10), in der eine Vielzahl von Teststreifen aufbewahrt wird, in einem Innenraum des Behälters ersetzbar aufzunehmen, und der einen Streifenauslass (101) enthält, durch den ein Teststreifen (S) ausgegeben wird;
eine Streifenladeeinheit (200), die in dem Behälter angeordnet ist, um als Reaktion auf eine Bedienung eines Benutzers linear beweglich zu sein, wobei die Streifenladeeinheit einen Abschnitt eines Teststreifens, der durch eine Zufuhröffnung (11) der Streifenkartusche (10) zugeführt wird, zu dem Streifenauslass (101) ausgibt, um in einem Zuckermessmodus geladen zu werden; und
ein Kartuschenöffnungs-/-schließmodul (400), das innerhalb des Behälters angeordnet ist, um mit einem Betrieb der Streifenladeeinheit (200) zusammenzuwirken, um die Zufuhröffnung (11) der Streifenkartusche (10) zu öffnen und zu schließen, **dadurch gekennzeichnet, dass:**
das Kartuschenöffnungs-/-schließmodul (400) Folgendes beinhaltet: eine drehbare Hülse (410), die sich zusammenwirkend mit einer linearen Bewegung der Streifenladeeinheit (200) dreht und bewegt; und
eine Öffnungs-/Schließabdeckung (430), die durch eine Drehbewegung der drehbaren Hülse (410) gedrückt oder aus einem gedrückten Zustand gelöst wird, um die Zufuhröffnung (11) der Streifenkartusche (10) zu öffnen und zu schließen,
wobei eine Trägerwelle (420) in einen Innenraum der drehbaren Hülse (410) in Richtung einer Mittelachse eingepasst ist, und die drehbare Hülse (410) mit einer äußeren Umfangsfläche der Trägerwelle (420) gekoppelt ist, sodass die drehbare Hülse (410) um die Mittelachse drehbar ist.

2. Integriertes Blutzuckermessgerät nach Anspruch 1, wobei ein Druckvorsprung (411) von einer äußeren Umfangsfläche der drehbaren Hülse (410) hervorsteht, um die Öffnungs-/Schließabdeckung (430) zu drücken,
wobei die Öffnungs-/Schließabdeckung (430) montiert ist, um in einer Richtung von oben nach unten linear beweglich zu sein, um die Zufuhröffnung (11) der Streifenkartusche (10) zu öffnen und zu schließen, und durch eine elastische Feder (240) in eine Richtung des Kontakts mit der drehbaren Hülse (410) elastisch nach oben getragen wird.

3. Integriertes Blutzuckermessgerät nach Anspruch 1, wobei die Streifenladeeinheit (200) Folgendes enthält:
einen Betätigungsknopf (210), der mit dem Behälter (100) verbunden ist, um auf einer Fläche des Behälters freigelegt zu sein und linear beweglich zu sein, sodass der Betätigungsknopf durch den Benutzer betätigt wird;
einen beweglichen Körper (220), der mit dem Betätigungsknopf (210) gekoppelt ist, um sich linear, fest verbunden mit dem Betätigungsknopf zu bewegen; und
eine Ladeplatte (230), die fest verbunden mit dem Betätigungsknopf (210) linear beweglich ist, um einen Teststreifen (S), der durch die Zufuhröffnung (11) der Streifenkartusche (10) zugeführt wird, durch Schieben des Teststreifens in den Streifenauslass (101) zu laden.

4. Integriertes Blutzuckermessgerät nach Anspruch 3, wobei die drehbare Hülse (410) eine Nockennut (412) aufweist und der bewegliche Körper (220) einen Nockenvorsprung (221) aufweist, der der Nockennut (412) entspricht, wobei die Nockennut (412) und der Nockenvorsprung (221) miteinander in Eingriff stehen, um eine Nockenstruktur bereitzustellen, und die drehbare Hülse (410) konfiguriert ist, um sich über die Nockenstruktur als Reaktion auf eine lineare Bewegung des beweglichen Körpers (220) zu drehen und zu bewegen.

5. Integriertes Blutzuckermessgerät nach Anspruch 3, wobei in einem Zustand, in dem das Kartuschenöffnungs-/-schließmodul (400) die Zufuhröffnung (11) der Streifenkartusche (10) geöffnet hat, die Ladeplatte (230) sich linear bewegt, um einen Teststreifen (S) zu schieben, wodurch die Zufuhröffnung (11) der Streifenkartusche (10) geschlossen wird.

6. Integriertes Blutzuckermessgerät nach Anspruch 3, wobei eine Bewegungsbegrenzungsführung (180), die die Streifenladeeinheit (220) führt, in dem Behälter (100) bereitgestellt ist, wobei die Bewegungsbegrenzungsführung (180) verhindert, dass die Streifenladeeinheit (200) in eine Referenzposition zurückkehrt, bevor ein linearer Bewegungsprozess eines Ladens eines Teststreifens abgeschlossen ist.

7. Integriertes Blutzuckermessgerät nach Anspruch 6, wobei die Bewegungsbegrenzungsführung (180) Folgendes enthält:
eine Bewegungsbahn-Führungsschiene (181), die die Streifenladeeinheit (200) entlang einer linearen Bewegungsbahn zum Laden eines Teststreifens führt; und
eine Rücklaufbahn-Führungsschiene (182), die die Streifenladeeinheit (200) entlang einer Rücklaufbewegungsbahn führt,
wobei die Bewegungsbahn-Führungsschiene (181) eine Vielzahl von Rastzähnen (183) aufweist, um zu verhindern, dass die Streifenladeeinheit (200) in eine Ausgangsposition zurückkehrt, und die Streifenladeeinheit einen Führungsvorsprung (250) aufweist, der in die Bewegungsbahn-Führungsschiene (181) und die Rücklaufbahn-Führungsschiene (182) einzuführen und von diesen zu führen ist.

8. Integriertes Blutzuckermessgerät nach einem der Ansprüche 1 bis 7, wobei in dem Behälter (100) eine Ladesperrvorrichtung (500) bereitgestellt ist, wobei die Ladesperrvorrichtung (500) einen Betrieb der Streifenladeeinheit (200) in einem Zustand verhindert, in dem die Streifenkartusche (10) nicht in dem Behälter (100) aufgenommen ist.

9. Integriertes Blutzuckermessgerät nach Anspruch 8, wobei die Ladesperrvorrichtung (500) Folgendes enthält:
einen Sperrblock (510), der innerhalb des Behälters (100) angeordnet ist, um linear beweglich zu sein, und als Reaktion auf ein Einführen der Streifenkartusche (10) mit der Streifenkartusche in Eingriff ist, um sich linear in eine Richtung zu bewegen, in die die Streifenkartusche eingeführt wird; und
ein elastisches Element (520), das eine elastische Kraft auf den Sperrblock (510) ausübt, sodass sich der Sperrblock elastisch in eine Richtung entgegen der Richtung bewegt, in der die Streifenkartusche (10) eingeführt wird,
wobei ein Betrieb der Streifenladeeinheit (200) abhängig von einer linearen Bewegung des Sperrblocks (510) blockiert oder freigegeben wird.

10. Integriertes Blutzuckermessgerät nach einem der Ansprüche 1 bis 7, ferner umfassend eine Auswurfeinheit (300), die in dem Behälter (100) angeordnet ist, um durch eine Druckbedienung des Benutzers funktionsfähig zu sein, um einen Teststreifen (S), der von der Streifenladeeinheit (200) geladen wird, aus dem Streifenauslass (101) auszugeben und zu entfernen.

11. Integriertes Blutzuckermessgerät nach einem der Ansprüche 1 bis 7, wobei der Behälter (100) mit einem Lanzettenanschluss (130) bereitgestellt ist, an dem eine Lanzettenvorrichtung abnehmbar angebracht ist.

## Revendications

1. Glucomètre sanguin intégré comprenant :
un récipient (100) conçu pour recevoir de manière échangeable une cartouche de bandelettes (10), dans lequel une pluralité de bandelettes de test est stockée, dans un espace interne du récipient, et comprenant une sortie de bandelette (101) par laquelle une bandelette de test (S) est déchargée ;
une unité de chargement de bandelette (200) disposée dans le récipient de façon à être mobile linéairement en réponse à une manipulation d'un utilisateur, ladite unité de chargement de bandelette déchargeant une partie d'une bandelette de test, alimentée par une ouverture d'alimentation (11) de la cartouche de bandelettes (10), à la sortie de la bandelette (101) devant être chargée dans un mode de mesure du glucose ; et
un module d'ouverture/fermeture de cartouche (400) disposé à l'intérieur du récipient de façon à fonctionner de concert avec une opération de l'unité de chargement de bandelette (200) pour ouvrir et fermer l'ouverture d'alimentation (11) de la cartouche de bandelettes (10), **caractérisé en ce que** :
le module d'ouverture/fermeture de cartouche (400) comprend :
un manchon rotatif (410) tournant et se déplaçant de concert avec un déplacement linéaire de l'unité de chargement de bandelette (200) ; et
un couvercle d'ouverture/fermeture (430) étant pressé ou libéré d'un état pressé par un mouvement de rotation du manchon rotatif (410) pour ouvrir et fermer l'ouverture d'alimentation (11) de la cartouche de bandelettes (10),
un arbre support (420) étant introduit dans un espace interne du manchon rotatif (410) suivant la direction d'un axe central, et le manchon rotatif (410) étant couplé à une surface circonférentielle externe de l'arbre support (420) de sorte que le manchon rotatif (410) puisse tourner autour de l'axe central.

2. Glucomètre sanguin intégré selon la revendication 1, une saillie de pression (411) faisant saillie depuis une surface circonférentielle externe du manchon rotatif (410) pour presser le couvercle d'ouverture/fermeture (430),
le couvercle d'ouverture/fermeture (430) étant monté de façon à être mobile linéairement dans une direction de haut en bas pour ouvrir et fermer l'ouverture d'alimentation (11) de la cartouche de bandelettes (10), et étant supporté élastiquement vers le haut suivant une direction de contact avec le manchon rotatif (410) au moyen d'un ressort élastique (240).

3. Glucomètre sanguin intégré selon la revendication 1, ladite unité de chargement de bandelette (200) comprenant :
un bouton de commande (210) relié au récipient (100) de façon à être exposé sur une surface du récipient et de façon à être mobile linéairement, de sorte que le bouton de commande soit actionné par l'utilisateur ;
un corps mobile (220) couplé au bouton de commande (210) de façon à se déplacer linéairement, solidairement avec le bouton de commande ; et
une plaque de chargement (230) mobile linéairement, solidairement avec le bouton de commande (210), pour charger une bandelette de test (S), délivrée par l'ouverture d'alimentation (11) de la cartouche de bandelettes (10), dans la sortie de bandelette (101) en poussant la bandelette de test.

4. Glucomètre sanguin intégré selon la revendication 3, ledit manchon rotatif (410) comportant une rainure de came (412) et ledit corps mobile (220) comportant une saillie de came (221) correspondant à la rainure de came (412), ladite rainure de came (412) et ladite saillie de came (221) s'engageant l'une avec l'autre pour fournir une structure de came, et ledit manchon rotatif (410) étant conçu pour tourner et se déplacer par le biais de la structure de came en réponse à un déplacement linéaire du corps mobile (220).

5. Glucomètre sanguin intégré selon la revendication 3, dans un état dans lequel le module d'ouverture/fermeture de cartouche (400) a ouvert l'ouverture d'alimentation (11) de la cartouche de bandelettes (10), ladite plaque de chargement (230) se déplaçant linéairement pour pousser une bandelette de test (S), fermant ainsi l'ouverture d'alimentation (11) de la cartouche de bandelettes (10).

6. Glucomètre sanguin intégré selon la revendication 3, un guide de limitation de déplacement (180) qui guide l'unité de chargement de bandelette (220) étant pourvu dans le récipient (100), le guide de limitation de déplacement (180) empêchant l'unité de chargement de bandelette (200) de revenir à une position de référence avant qu'un processus de déplacement linéaire de chargement d'une bandelette de test ne soit terminé.

7. Glucomètre sanguin intégré selon la revendication 6, ledit guide de limitation de déplacement (180) comprenant :
un rail de guidage de trajectoire de déplacement (181) qui guide l'unité de chargement de bandelette (200) le long d'une trajectoire de déplacement linéaire pour le chargement d'une bandelette de test ; et
un rail de guidage de trajectoire de retour (182) qui guide l'unité de chargement de bandelette (200) le long d'une trajectoire de déplacement de retour,
ledit rail de guidage de trajectoire de déplacement (181) comportant une pluralité de dents à cliquet (183) destinée à empêcher le retour de l'unité de chargement de bandelette (200) à une position initiale, et ladite unité de chargement de bandelette comportant une saillie de guidage (250) destinée à être insérée dans le rail de guidage de trajectoire de déplacement (181) et le rail de guidage de trajectoire de retour (182) et guidée par ceux-ci.

8. Glucomètre sanguin intégré selon l'une des revendications 1 à 7, un dispositif de verrouillage de chargement (500) étant pourvu dans le récipient (100), le dispositif de verrouillage de chargement (500) empêchant un fonctionnement de l'unité de chargement de bandelette (200) dans un état dans lequel la cartouche de bandelettes (10) n'est pas reçue dans le récipient (100).

9. Glucomètre sanguin intégré selon la revendication 8, ledit dispositif de verrouillage de chargement (500) comprenant :
un bloc de verrouillage (510) disposé à l'intérieur du récipient (100) de façon à être mobile linéairement et, en réponse à une insertion de la cartouche de bandelettes (10), est engagé avec la cartouche de bandelettes pour se déplacer linéairement dans une direction dans laquelle la cartouche de bandelettes est insérée ; et
un élément élastique (520) appliquant une force élastique sur le bloc de verrouillage (510) de sorte que le bloc de verrouillage se déplace élastiquement dans une direction opposée à la direction dans laquelle la cartouche de bandelettes (10) est insérée,
une opération de l'unité de chargement de bandelette (200) étant bloquée ou débloquée, en fonction d'un déplacement linéaire du bloc de verrouillage (510).

10. Glucomètre sanguin intégré selon l'une des revendications 1 à 7, comprenant en outre une unité d'éjection (300) disposée dans le récipient (100) de façon à pouvoir être actionnée par une manipulation par pression de l'utilisateur pour décharger et retirer une bandelette de test (S), chargée par l'unité de chargement de bandelette (200), depuis la sortie de bandelette (101).

11. Glucomètre sanguin intégré selon l'une des revendications 1 à 7, ledit récipient (100) étant pourvu d'un raccord de lancette (130) auquel un dispositif de lancette est fixé de manière amovible.
